Europäisches Patentamt

European Patent Office

Office européen des brevets

(11) Publication number: **0 157 574**
**B1**

(12) EUROPEAN PATENT SPECIFICATION

(45) Date of publication of patent specification: **06.12.89**

(21) Application number: **85302070.9**

(22) Date of filing: **26.03.85**

(51) Int. Cl.⁴: **C 12 N 5/00, C 12 P 21/00**

(54) Antitetanic antibody producing human-human hybridoma and method of producing the same.

(30) Priority: **28.03.84 JP 60061/84**

(43) Date of publication of application:
**09.10.85 Bulletin 85/41**

(45) Publication of the grant of the patent:
**06.12.89 Bulletin 89/49**

(84) Designated Contracting States:
**BE DE FR GB NL SE**

(56) References cited:

**PROC. NATL. ACAD. SCI. USA, vol. 79, November 1982, pages 6651-6655, US; D. KOZBOR et al.: "Human hybridomas constructed with antigen-specific Epstein-Barr virus-transformed cell lines"**

**THE JOURNAL OF IMMUNOLOGY, vol. 133, no. 6, December 1984, pages 3001-3005, The American Association of Immunologists, US; D. KOZBOR et al.: "A human hybrid myeloma for production of human monoclonal antibodies"**

(73) Proprietor: **GREEN CROSS CORPORATION**
**15-1, Imabashi-1-chome Higashi-ku Osaka-shi Osaka 541 (JP)**

(72) Inventor: **Amatsuji, Yasuo**
**6-2, Nagaomotomachi-1-chome Hirakata-shi (JP)**
Inventor: **Ishikawa, Hideyuki**
**33-402, Senriokanaka Suita-shi (JP)**
Inventor: **Arimura, Hirofumi**
**18-1-401, Uenosaka-2-chome Toyonaka-shi (JP)**
Inventor: **Nishida, Masayuki**
**12-1, Kayogaoka-2-chome Nagaokakyo-shi (JP)**
Inventor: **Suyama, Tadakazu**
**3-7, Matsuigaoka-4-chome Tanabecho Tsuzuki-gun Kyoto (JP)**

(74) Representative: **Harrison, David Christopher et al**
**MEWBURN ELLIS & CO 2/3 Cursitor Street London EC4A 1BQ (GB)**

Courier Press, Leamington Spa, England.

# EP 0 157 574 B1

(56) References cited:
BIOLOGICAL ABSTRACTS, vol. 77, no. 10, 1984, no. 75764, Philadelphia, PA, US; D. KOZBOR et al.: "Human anti-tetanus toxoid monoclonal antibody secreted by Epstein-Barr virus-transformed human B cells fused with murine mycloma", & HYBRIDOMA 1(3): 323-328, 1982(Recd. 1983)

THE JOURNAL OF IMMUNOLOGY, vol. 127, no. 4, October 1981, pages 1275-1280, The American Association of Immunologists, US; D. KOZBOR et al.: "Requirements for the establishment of high-titered human monoclonal antibodies against tetanus toxoid using the Epstein-Barr virus technique" PROC. NATL. ACAD. SCI. USA, vol. 80, October 1983, pages 6376-6380, US; J. W. LARRICK et al.: "Characterization of human hybridomas secreting antibody to tetanus toxoid"

**Description**

This invention relates to a novel human-human hybridoma. More particularly, it relates to a human-human hybridoma having antitetanic antibody producing ability.

The human-human hybridoma according to this invention can be utilized for the production of a monoclonal antibody against Clostridium tetani. Further, since the cell line is of human origin, the antibody produced by said hybridoma causes no trouble due to antigenicity. Thus, a great therapeutic effect can be produced by this invention.

It has been known that antitetanus antibody has an effect of preventing the onset of tetanus by administration to persons infected with Clostridium tetani. Further, it has been revealed recently that the administration of tetanus antibody can prevent not only the onset of tetanus but also the infection itself. Accordingly, the antibody is expected to make a great medical contribution.

There are in general two kinds of antibodies, namely polyclonal antibodies and monoclonal ones. Recently a technology is attracting attention wherein monoclonal antibodies are prepared by using a lymphoblastoid cell line having proliferative ability.

This monoclonal antibody is a single antibody which reacts against only one kind of antigenic determinant. Preparation thereof can be conducted at present by either cell fusion method or transformation method. In both methods, there are prepared cells having concurrently both proliferative ability and antibody producing ability. The former method comprises fusion of B-cells of an immunized donor with B cell line in vitro and the latter comprises infecting B-cells of an immunized donor with lymphotropic viruses such as Epstein Barr virus to transform the former cells into immortalized cells.

These methods of preparation are as yet in unripe stage and have a number of unsolved problems including improving the antibody-producing capacity, safety measures against the use of toxic antigen, and overcoming the difficulty in removal of contaminating antigen by purification. Thus, there exist many difficulties to be overcome before they can be put to practical use. Namely, in the cell fusion method a satisfactory antibody-producing hybridoma has not yet been obtained because of low fusion efficiency and low proportion of antitetanic antibody producing cells present in the whole lymphocytes, whereas the transformation method has a drawback in that although established cells can be obtained in a very high probability the cloning efficiency is extremely low (as low as only several percent.)

The problems relating to the antigenicity of antibodies produced can be solved by using a cell line of an animal which does not give rise to immunoreaction with human beings. It is already known in general that safety can be maintained by using a cell line of human origin. However in this case, fully satisfactory results have not yet been obtained with respect to antibody-producing ability and proliferative ability.

Kozbor et al, Proc. Natl. Acad. Sci., (Nov. 1982), 79, 6651—6655, describe the construction of an antitetanic antibody producing hybridoma in which an antitetanic antibody producing human lymphoblastoid cell transformed with Epstein-Barr virus is fused with a proliferating human, HAT sensitive, ouabain resistant B cell line.

The proliferating B cell line is prepared from a human B cell line GM 1500 rendered resistant to 6-thioguanine (and therefore HAT sensitive) by a mutagenesis treatment using ethyl methane sulphonate; see Croce et al, Nature, (Dec. 1980), 288, 488—489. The HAT sensitive B cell line is rendered resistant to ouabain by a mutagenesis treatment using γ-radiation (200—R). Subsequent treatment, including subculturing in gradually increasing concentrations of ouabain, yields a cell line KR4 found to be resistant to 0.5mM ouabain 5 months after mutagenesis. However, such mutagenesis treatments are considered likely to cause mutation in genes other than that of ouabain.

Attempts were also made to fuse human 6-thioguanine resistant B cell lines not subjected to further mutagenesis or subjected to alternative mutagenesis treatments but the frequency of hybrid formation was very low.

As a part of an effort for establishing an antitetanic antibody producing cell line which causes no difficulties mentioned above and can be produced in a large scale, we have made extensive studies on a human-human hybridoma which is free from the problem of antigenicity. As a result, it has been found out that a human-human hybridoma having a high proliferative ability and which is free from the problem of antigenicity can be obtained, as a means for overcoming the various difficulties involved in antibody production mentioned above, by preparing, in a special manner as described below, a proliferative human B cell line having HAT (hypoxanthine, aminopterin, thymidine) sensitivity and ouabain resistance, a novel parent cell, and fusing the proliferative human B cell line with an antitetanic antibody producing lymphoblastoid cell line of human origin transformed with a lymphotropic virus such as Epstein Barr virus. This invention has been accomplished on the basis of above finding.

This invention provides a human-human hybridoma for antitetanic antibody production which has an excellent proliferative ability and is free from the problem of antigenicity, and provides a human-human hybridoma obtained by fusing the human, HAT sensitive, ouabain resistant B cell line with a human, antitetanic antibody producing B limphoblastoid cell transformed with a lymphotropic virus.

The cell fusion in this invention is thus conducted by using a cell having a proliferative ability and a cell having an antibody producing ability.

The antibody producing cell used in this invention is a human, antitetanic antibody producing lymphoblastoid transformed with a lymphotropic virus.

EP 0 157 574 B1

The above-mentioned lymphoblastoid cell can be prepared by known methods, for example in the following manner.

The tetanus antigen used to obtain said lymphoblastoid cell is an exotoxin which is a protein having a molecular weight of about 150,000 and an isoelectric point of 5.I produced by Clostridium tetani. It can usually be prepared by purifying a commercially available detoxicated toxoid or a culture fluid conditioned by Clostridium tetani. It is also possible to subject the tetanus antigen thus obtained to a heat treatment in the presence of a protein denaturing agent to enhance its antigenicity. The antigen is preferably further purified for immunization.

A B cell may then be stimulated with above-mentioned tetanus antigen for antibody production and this stimulation may be conducted either in vivo or in vitro. Stimulation in vivo can be conducted by known methods. For example, the intended human, antitetanic antibody producing B cells can be obtained by inoculating a human being with the tetanus antigen intracutaneously 2 to 3 times collecting the blood several days after final immunization, then recovering B cells from the blood obtained above followed by purification thereof. The stimulation with tetanus antigen in vitro can be conducted, for example, by bringing a suitable amount of purified antigen into contact with the B cells at 30 to 40°C for 10 to 50 hours, generally in a culture medium. The separation of the antitetanic antibody producing B cells is preferably conducted by means of Ficoll-Conray density gradient centrifugation.

The B cell thus obtained is then transformed into the immortalized cell by using a lymphotropic virus.

Lymphotropic viruses, such as Epstein Barr virus, are used in the transformation. Such viruses are known to be capable of transforming a normal cell into an immortalized one [Nature 269, 420—422 (1977)]. An amount of virus sufficient for enabling cell transformation may be prepared beforehand by using Epstein Barr virus derived from the marmoset cell line B 95—8 (free from mycoplasmas; obtained as a supernatant from B 95—8). A suitable amount of the virus thus prepared is added dropwise to a medium conditioned by B cells and the virus is contacted with the B cells at 37°C for 5 to 20 days. The cultivation of B cells is conducted, for example, in 5% fetal bovine serum at 37°C. The cultivation may also be conducted in a culture medium supplemented with glutamine.

The immortalized B cells obtained thus by transformation are subcultured and those cells which produce antibodies against tetanus antigen are selected out of the subcloned cells and concentrated.

The antitetanic antibody producing cells can be traced by such methods as, for example, PHA, EIA and RIA.

A human, HAT sensitive, ouabain resistant B cell is used in this invention as the cell having proliferative ability. The said human, HAT sensitive, ouabain resistant B cell line is prepared as follows. An autogenetic HAT sensitive cell is obtained by gradually increasing the concentration of 6-thioguanine in the medium, optionally over a long time, in cultivation of human B cell line. The HAT sensitive, B cell line is rendered ouabain resistant by increasing the concentration of ouabain (such increase may be gradual) in the culturing medium of the HAT sensitive B cell line and collecting the cells which survive. By this procedure, it is possible to make the cells acquire a marker function, that is, ouabain resistance, without affecting properties inherent in the cells HAT sensitivity, fusion efficiency, proliferative ability and so forth), whereby the selection of intended cells is facilitated.

Then, cell fusion is conducted by using the human, antitetanic antibody producing B cells having proliferative ability owing to transformation, that is, human antitetanic antibody producing lymphoblastoid cells, and the human, HAT sensitive, ouabain resistant B cell line obtained above. The cell fusion is conducted in a manner known per se. One example thereof comprises reacting immortalized cells with the aforementioned lymphoblastoid cell capable of producing antibodies in the presence of polyethylene glycol. A suitable mixing ratio of the cells is 1 to 10 lymphoblastoid cells relative to one immortalized cell.

The fused cells thus obtained are subjected to selection using a medium supplemented with HAT and ouabain. The selection can be conducted based on the following ground. The HAT sensitivity means the property of a cell that it cannot grow or proliferate in the presence of HAT in the medium. The ouabain resistance of a cell means that the cell can proliferate even in the presence of a high concentration of ouabain. It is also known that the fusion of an ouabain resistant cell and a normal one results in an ouabain resistant cell. Accordingly, when human, HAT sensitive, ouabain resistant B cell line and human, antitetanic antibody producing lymphoblastoid cells (the lymphoblastoid cells being devoid of both HAT sensitivity and ouabain resistance) are subjected to cell fusion and when both HAT and ouabain are present in the medium, neither unfused B cell line nor unfused lymphoblastoid cell can proliferate, the former because of HAT sensitivity and the latter because of not being ouabain resistant, whereas successfully fused cells can proliferate because of being ouabain resistant. It is possible thus to select the fused cells alone.

The antitetanic antibody producing human-human hybridoma can be obtained by further screening and cloning the cells producing the intended antitetanic antibody by a known method.

Since the hybridoma thus obtained is of human origin, the antibody produced therefrom causes no problem due to antigenicity against human beings. Moreover, an antibody producing cell can be obtained which is excellent in antibody producing ability and particularly in proliferative ability as compared with those obtained by simply fusing human HAT sensitive B cell line with human, antitetanic antibody producing B cells or by merely transforming human, antitetanic antibody producing lymphoblastoid cells transformed with Epstein Barr virus.

4

By proliferating the hybridoma in a growth medium, monoclonal antibodies can be produced continuously and then recovered from the medium.

This invention will be described in more detail below with reference to Examples, but it is not limited thereto.

Example 1

Each of healthy, adult volunteers was subjected to a booster immunization of tetanus toxoid (0.5 ml). Blood was collected at the about 4th day from the injection. Lymphocytes were isolated from the peripheral blood collected above by means of Ficoll-Conray density gradient centrifugation and then T cells were removed therefrom by means of E-rosette formation to obtain B cells alone. The B cells were then cultivated in an RPMI 1640 supplemented with 15% fetal bovine serum and 2 mM glutamine medium (cell density: $1 \times 10^6$ cells/ml). Meanwhile, B 95—8 cells (Marmoset established lymphocyte cell) were cultivated in an RPMI 1640 supplemented with 20% fetal bovine serum medium and the supernatant culture fluid was collected after several days of cultivation to be used as Epstein Barr virus liquid (concentration: $10^5$ transforming dose/ml). One ml of the above-mentioned medium conditioned by B cells was mixed with 0.2 ml of the Epstein Barr virus liquid to effect infection and then subjected to static culture for three weeks in a medium (RPMI 1640 supplemented with 20% fetal bovine serum) under 5% $CO_2$ atmosphere to proliferate the cells. Recovery of antibody producing cells from the proliferated ones was conducted by limiting dilution culture method while checking by ELISA method. Thus, subcultured cells having antitetanic antibody producing ability were obtained.

On the other hand, human, HAT sensitive, B cell line were prepared in the following manner. Human B cell line in their logarithmic growth phase were cultured with a medium (RPMI 1640 supplemented with 10% fetal bovine serum) containing 0.05 μg/ml of 6-thioguanine. Then surviving cells were cultured in gradually increasing concentrations of 6-thioguanine, and after 3 months, the cells were found to be resistant to 6-thioguanine and showed high sensitivity to HAT.

Human HAT sensitive, ouabain resistant B cell line were prepared in the following manner.

The above-mentioned human, HAT sensitive B cell line in their logarithmic growth phase was diluted in a medium (RPMI 1640 supplemented with 10% fetal bovine serum) to a density of 8 cells/ml. The diluted liquid was dispensed onto a 96-well microplate at a rate of 100 μl/well to give a number of cells per well of 0.8. The plate was incubated at 37°C under 5% $CO_2$ atmosphere for about 2 weeks. After the period, proliferation of cells was observed to be occurring in a cloning efficiency of 30 to 40%. The most proliferative cells among these were adjusted to a cell density of about $10^6$ cells/ml, the resulting culture fluid was mixed with $10^{-7}$ M, ouabain, and cultivation was conducted in the mixed medium. Similar medium exchange was repeated 2 to 3 times and finally cultivation was conducted in a medium containing $10^{-6}$ M of ouabain. Thereafter, cultivation was conducted in a medium containing no ouabain and a very small number of cells which had survived were proliferated. After about 10 days the proliferated cells were washed by centrifugation, suspended in a medium containing $10^{-6}$ M of ouabain to a density of $10^5$ cells/ml and the suspension was dispensed onto a 96-well microplate at a rate of 100 μl/well. The plate was incubated for about one month, the medium being exchanged 2 times a week. Thus, cells were obtained which can proliferate even in the presence of $10^{-6}$ M ouabain.

The HAT sensitive, ouabain resistant B cell line ($1.5 \times 10^6$ cells) and the aforementioned lymphoblastoid cells ($3 \times 10^6$ cells) were mixed, and the mixture was further mixed with 0.3 ml of 50% polyethylene glycol (PEG 1500, mfd by BDH) and was incubated at 37°C for 2 minutes to effect cell fusion. After washed by centrifugation, the cells were adjusted to a density of $1 \times 10^6$ cells/ml with an RPMI 1640 supplemented with 10% fetal bovine serum medium and resulting liquid was dispensed onto a 96-well microplate at a rate of 100 μl/well. A HAT + ouabain selective medium [final concentration: hypoxanthine 13.61 mg/l, aminopterin 0.18 mg/l, thymidine 3.88 mg/l, ouabain $0.5 \times 10^{-6}$ M] was added 24 hours after the PEG treatment and cultivation was then conducted for 2 weeks.

As a result, proliferation of cells was observed in 20 wells among 43 wells. The antitetanic antibody activities in the supernatants of these culture fluids were screened by ELISA method. Of 12 wells which showed a high antibody production, the culture fluid contained therein was adjusted to a density of 8 cells/ml by dilution with an RPMI 1640 supplemented with 10% fetal bovine serum. The resulting fluid was dispensed onto a 96 well-microplate at a rate of 100 μl/well to give a number of cells per well of 0.8 (limiting dilution culture method). Selection was conducted by checking the antibody production by means of ELISA method to obtain an established human-human hybridoma [In the above ELISA method was used peroxidaselabelled anti-human IgA + IgG + IgM goat antibody (mfd. by KPL Co.)].

It was revealed that the human-human hybridoma thus obtained partook well of the nature of the respective parents as to the proliferative and antibodyproducing ability and was excellent in these properties as compared with hybridomas (normal B cells and HAT sensitive B cell line) or transformed cells obtained by prior methods.

Experimental Example 1

Two strains of hybridomas (10C, 10D) obtained in Example 1 were examined for their number of chromosomes (Table 1). The values in the Table show the number of cells having the indicated number of chromosomes.

As shown in Table 1, it was revealed that the hybridomas had several times (up to thirty times) more chromosomes than their parent cells, lymphoblastoid cells and HAT sensitive, ouabain resistant B cell line (each having 46 chromosomes) had. Thus, the fact that said cells are hybridomas differing from the parent cells is suggested also from the number of chromosomes.

Experimental Example 2

The properties of the established human-human hybridoma obtained in Example 1 were examined and the results were shown in Table 2. "Hybridoma formation or transformation efficiency" was indicated by the number of hybridomas or transformed cells emerging per $1 \times 10^6$ parent cells. "Cloning efficiency", used as an index signifying the proliferative ability, indicates the probability of proliferatable hybridomas appearing in the whole emerging hybridomas. As a control were used human B cell line and human, antitetanic antibody producing B cells, which are both the parent cells of the present hybridoma, as well as hybridoma between B cell line and normal B cells and lymphoblastoid cell transformed with Epstein Barr virus.

As shown in Table 2, it was revealed that the established human-human hybridoma of this invention can become a very effective antitetanic antibody producing cell with a good emerging rate as well as a good proliferative ability. Moreover, the hybridoma of this invention causes no problem due to antigenicity because it is of human origin.

Table 1

| Cell \ Number of chromosomes | 30 | 32 | 35 | 46 | 49 | 52 | 54 | 57 | 59 | 62 | 63 | 69 | 70 | 75 |
|---|---|---|---|---|---|---|---|---|---|---|---|---|---|---|
| B cell | | | | 12 | | | 1 | | | | | | | |
| HAT sensitive, ouabain resistant B cell line | 1 | 1 | 1 | 7 | 1 | | | | | | | | | |
| Hybridoma 10C | | | | | | 3 | | 3 | 1 | 2 | | 2 | | |
| Hybridoma 10D | | | | 1 | | | 1 | 1 | | | 4 | 1 | 1 | 2 |

EP 0 157 574 B1

Table 2

| Cell | Hybridoma formation or transformation efficiency (per $1 \times 10^6$ parent cells) | Cloning efficiency |
|---|---|---|
| HAT sensitive, ouabain resistant B cell line | | 30 to 40 % |
| Hybridoma (normal B cells and HAT sensitive B cell line) | 1 to 5 cells | 30 to 40 % |
| Lymphoblastoid cells (transformed with Epstein Barr Virus) | 20 to 30 cells | 1 % |
| Hybridoma (this invention) | 43 cells | 47 % |

EP 0 157 574 B1

### Experimental Example 3

The monoclonal antibody obtained by this invention was tested for its class and specificity. The sample antibody used in the test was produced by human-human hybridoma cell line used in Experimental Example 1. The class of monoclonal antibody was determined (Enzyme Linked Immunosorbent Assay) using antibody against various classes of human immunoglobulin. The specificity of monoclonal antibody was determined by assaying the reactivity against the fragment of tetanus toxin separated by SDS-PAGE (polyacrylamidegel) electrophoresis using the method of Western Blotting. The results are shown in Table 3. The class of monoclonal antibody was gamma and kappa for 10C cell, and mu and lambda for 10D cell. The specificity of monoclonal antibody produced by the 10C cell or 10D cell have recognized β fragment proposed by Helting et al or α fragment proposed by Matsuda et al, respectively.

Table 3

| cell | the class of IgG | Antigenspecificity |
|---|---|---|
| 10C | gamma, kappa, lambda, | β-fragment of tetanus toxin |
| 10D | gamma, mu, kappa, lambda | α-fragment of tetanus toxin |
| HAT sensitive | gamma, kappa | |
| Ouabain resistant | | none |

### Experimental Example 4

The amount of antibody which human-human hybridoma cell line used in Experimental Example 1 produced, by means of ELISA method using human IgG and IgM as standard, was measured. Consequently, the amount produced per $10^6$ cells per day was 5 g/ml on 10C cell and 1 g/ml on 10D cell.

## Claims

1. A method of producing an antitetanic antibody producing human-human hybridoma, which method comprises the steps of
   a) preparing a human, HAT sensitive, ouabain resistant B cell line, and
   b) fusing the human, HAT sensitive, ouabain resistant B cell line with a human, antitetanic antibody-producing lymphoblastoid cell which has been transformed with a lymphotropic virus,
   characterized in that step (a) is carried out by the steps of
   i) rendering a human B cell line resistant to 6-thioguanine, and consequently HAT sensitive, by cultivating the cell line in the presence of gradually increasing concentrations of thioguanine, and thereafter
   ii) rendering the HAT sensitive human B cell line resistant to ouabain by cultivating the said cell line in increasing concentrations of ouabain.

2. A method according to Claim 1, wherein the human, antitetanic antibody producing lymphoblastoid cell is prepared by stimulating a human lymphoblastoid cell with a tetanus antigen *in vivo* or *in vitro*.

3. A method according to Claim 2, wherein the stimulation *in vivo* is effected by inoculating a human being with a detoxicated tetanus antigen, and collecting the stimulated B cells.

4. A method according to Claim 2, wherein the stimulation *in vitro* is effected by bringing a detoxicated tetanus antigen into contact with human B cells in a culture medium, and collecting the stimulated B cells.

5. A method according to any preceding claim, wherein the lymphotropic virus is Epstein Barr virus.

6. A method according to any preceding claim, wherein the transformation has been conducted by bringing the human, antitetanic antibody producing B cell into contact with the lymphotropic virus in a culture medium conditioned by the said B cell.

7. A method according to any preceding claim, wherein the cell fusion is conducted by use of polyethylene glycol.

8. A method according to any preceding claim, wherein the cell fusion is conducted in a ratio of 1 to 10 cells of the said lymphoblastoid cells relative to one cell of the said HAT sensitive, ouabain resistant B cell line.

9. A human-human hybridoma capable of the formation of a monoclonal antibody against Clostridium tetani, produced by the method of any one of Claims 1 to 8.

10. A process for producing a monoclonal antibody against Clostridium tetani, which comprises cultivating a human-human hybridoma produced by the method of any one of Claims 1 to 8.

11. A monoclonal antibody produced by the method according to Claim 10.

**Patentansprüche**

1. Verfahren zur Herstellung von Antitetanus-Antikörperproduzierenden Mensch-Mensch-Hybridomen, das die folgenden Schritte umfaßt:

a) Herstellung einer HAT-sensitiven, Ouabain-resistenten menschlichen B-Zellinie; und

b) Fusion der HAT-sensitiven, Ouabain-resistenten menschlichen B-Zellinie mit einer Antitetanus-Antikörper-produzierenden menschlichen lymphoblastoiden Zelle, die mit einem lymphotropen Virus transformiert worden ist;

dadurch gekennzeichnet, daß Schritt a) wie folgt ausgeführt wird:

i) Herstellung einer gegen 6-Thioguanin resistenten und somit HAT-sensitiven menschlichen B-Zellinie durch Kultivierung der Zellinie in Gegenwart von schrittweise erhöhten Thioguanin-Konzentrationen; und

ii) Herstellung einer Ouabain-resistenten menschlichen B-Zellinie aus der HAT-sensitiven durch Kultivierung der Zellinie bei steigenden Ouabain-Konzentrationen.

2. Verfahren nach Anspruch 1, wobei die Antitetanus-Antikörper-produzierende menschliche lymphoblastoide Zelle durch Stimulierung einer menschlichen lymphoblastoiden Zelle mit einem Tetanus-Antigen in vivo oder in vitro hergestellt wird.

3. Verfahren nach Anspruch 2, wobei die in vivo-Stimulierung durch Impfung eines Menschen mit einem nicht-toxisch gemachten Tetanus-Antigen bewirkt wird und die stimulierten B-Zellen gesammelt werden.

4. Verfahren nach Anspruch 2, wobei die in vitro-Stimulierung in einem Kulturmedium durch In-Kontakt-Bringen eines nicht-toxisch gemachten Tetanus-Antigens mit menschlichen B-Zellen bewirkt wird und die stimulierten B-Zellen gesammelt werden.

5. Verfahren nach einem der vorhergehenden Ansprüche, wobei das lymphotrope Virus Epstein-Barr-Virus ist.

6. Verfahren nach einem der vorhergehenden Ansprüche, wobei die Transformation in einem durch die B-Zelle konditionierten Kulturmedium durch In-Kontakt-Bringen der Antitetanus-Antikörper-produzierenden menschlichen B-Zelle mit dem lymphotropen Virus durchgeführt wird.

7. Verfahren nach einem der vorhergehenden Ansprüche, wobei die Zellfusion mittels Polyäthylenglykol durchgeführt wird.

8. Verfahren nach einem der vorhergehenden Ansprüche, wobei die Zellfusion in einem Verhältnis von 1 bis 10 Zellen der lymphoblastoiden Zellen pro einer Zelle der HAT-sensitiven, Ouabain-resistenten B-Zellinie durchgeführt wird.

9. Mensch-Mensch-Hybridom, das einen monoclonalen Antikörper gegen Clostridium tetani bilden kann und nach dem Verfahren nach einem der Ansprüche 1 bis 8 hergestellt ist.

10. Verfahren zur Herstellung von monoclonalen Antikörpern gegen Clostridium tetani, das die Kultivierung eines nach dem Verfahren nach einem der Ansprüche 1 bis 8 hergestellten Mensch-Mensch-Hybridoms umfaßt.

11. Monoclonaler Antikörper, hergestellt nach dem Verfahren nach Anspruch 10.

**Revendications**

1. Procédé de préparation d'un anticorps antitétanique produisant un hybridome humain-humain, lequel procédé comprend les étapes consistant à:

a) préparer une lignée cellulaire B humaine résistant à l'ouabaïne, sensible au HAT, et

b) fusionner la lignée cellulaire B humaine résistant à l'ouabaïne et sensible au HAT avec une cellule lymphoblastoïde humaine, produisant des anticorps antitétaniques ayant été transformée par un virus lymphotrope, caractérisé en ce que l'étape a) comprend les étapes consistant à:

i) rendre une lignée cellulai re B humaine résistant à la 6-thioguanine, et par conséquent sensible au HAT, par culture de la lignée cellulaire en présence de concentrations progressivement croissantes de thioguanine, puis

ii) rendre la lignée cellulaire B humaine sensible au HAT, résistant à l'ouabaïne par culture de ladite lignée cellulaire dans des concentrations croissantes d'ouabaïne.

2. Procédé selon la revendication 1, dans lequel la cellule lymphoblastoïde humaine produisant des anticorps antitétaniques est préparée par stimulation d'une cellule lymphoblastoïde humaine avec un antigène du tétanos *in vivo* ou *in vitro*.

3. Procédé selon la revendication 2, dans lequel la stimulation *in vivo* est effectuée par inoculation à un être humain d'un antigène du tétanos biotransformé, et rassemblement des cellules B stimulées.

4. Procédé selon la revendication 2, dans lequel la stimulation *in vitro* est effectuée par mise en contact d'un antigène du tétanos biotransformé avec des cellules B humaines dans un milieu de culture, et rassemblement des cellules B stimulées.

5. Procédé selon l'une quelconque des revendications précédentes, dans lequel le virus lymphotrope est le virus d'Epstein Barr.

6. Procédé selon l'une quelconque des revendications précédentes, dans lequel la transformation a été réalisée par mise en contact de la cellule B humaine produisant des anticorps antitétaniques avec le virus lymphotrope dans un milieu de culture conditionné par ladite cellule B.

7. Procédé selon l'une quelconque des revendications précédentes, dans lequel la fusion cellulaire s'effectue par utilisation de polyéthylène glycol.

8. Procédé selon l'une quelconque des revendications précédentes, dans lequel la fusion cellulaire s'effectue selon un rapport de 1 à 10 desdites cellules lymphoblastoïdes à une cellule de ladite lignée cellulaire B résistant à l'ouabaïne et sensible au HAT.

9. Hybridome humain-humain capable de produire un anticorps monoclonal contre le Clostridium tetani, produit par le procédé de l'une quelconque des revendications 1 à 8.

10. Procédé pour la préparation d'un anticorps monoclonal actif contre le Clostridium tetani, qui comprend la culture d'un hybridome humain-humain produit par le procédé de l'une quelconque des revendications 1 à 8.

11. Anticorps monoclonal produit par le procédé de la revendication 10.